# EUROPEAN PATENT SPECIFICATION

(11) **EP 4 056 123 B1**
(45) Date of publication and mention of the grant of the patent: **07.01.2026**
(21) Application number: 20885677.3
(22) Date of filing: 06.11.2020
(51) Int. Cl.: A61B 8/08, A61B 8/00, A61B 5/00, A61B 8/12

(54) **THREE-DIMENSIONAL DIAGNOSTIC SYSTEM**
DREIDIMENSIONALES DIAGNOSESYSTEM
SYSTÈME DE DIAGNOSTIC TRIDIMENSIONNEL

(30) Priority: 06.11.2019 KR 20190141198
(43) Date of publication of application: 14.09.2022
(73) Proprietor: Daegu Gyeongbuk Institute Of Science and Technology, Daegu 42988 (KR)
(72) Inventor: HWANG, Jae Youn, Dalseong-gun Daegu 42988 (KR); KIM, Ji Hun, Changwon-si Gyeongsangnam-do 51167 (KR); LEW, Hah Min, Seoul 06370 (KR); LEE, Kyung Su, Daejeon 35284 (KR)
(74) Representative: Murgitroyd & Company
(86) International application number: PCT/KR2020/015460
(87) International publication number: WO 2021/091282

(56) References cited:
- JP-A- 2014 124 309
- JP-A- 2018 514 748
- JP-B2- 6 538 956
- KR-B1- 101 744 110
- KR-B1- 102 001 980
- US-A1- 2013 345 556
- US-A1- 2016 228 097
- US-A1- 2018 270 474

## Description

### Technical Field

A three-dimensional diagnosis system is disclosed.

### Background Art

There has been development of medical imaging systems capable of implementing three-dimensional images to diagnose a lesion such as cancer. Such imaging equipment is advantageous in that the treatment method is determined by inspecting the surface distribution or invasive depth of a tumor of a pre-operation or incised tissue according to the imaging technique applied to each imaging system. Recent endoscope-based imaging systems are required to acquire both surface information and depth information and to register the same, in order to three-dimensionally diagnose the lesion distribution on the surface of the target tissue and the lesion distribution inside the tissue.

US patent application nos. US 2013/345556 A1, US 2016/228097 A1, and US 2018/270474 A1 are useful for understanding the present invention.

### SUMMARY OF THE INVENTION

A three-dimensional diagnosis system according to claim 1 is disclosed.

### SUMMARY OF THE DISCLOSURE

### Technical Problem

An aspect according to an example is to provide a system capable of providing not only surface information of a lesion, but also depth direction information thereof.

An aspect according to an example is to provide a system capable of inspecting the distribution and invasive depth of a lesion.

An aspect according to an example is to provide a system capable of endoscopic optical imaging and ultrasound imaging with the same wide field of view.

An aspect according to an example is to provide a system capable of diagnosing and imaging a lesion distribution of a tissue surface through real-time spectral imaging and analysis.

An aspect according to an example is to provide a system capable of high-frequency imaging with the same field of view as optical imaging.

An aspect according to an example is to provide a system capable of sensing movements of an endoscope probe.

An aspect according to an example is to provide a system capable of stabilizing ultrasound images acquired in response to movements of an endoscope probe in real time.

An aspect according to an example is to provide a system capable of generating similar ultrasound images filling empty spaces between ultrasound images acquired based on ultrasound images acquired in response to movements of an endoscope probe.

Problems to be solved by examples are not limited to the above-mentioned problems, and other problems not mentioned herein will be clearly understood from the following description by those skilled in the art.

### Solution to Problem

In accordance with an aspect, a three-dimensional diagnosis system according to an example may include:
a probe configured to be positioned at an object to be diagnosed; a spectral image acquisition unit configured to acquire a spectral image with respect to surface information of the object through the probe; an ultrasound image acquisition unit configured to acquire an ultrasound image with respect to depth information of the object through the probe; a color image acquisition unit configured to acquire a color image of the object through the probe; an inertia measurement unit configured to detect movement of the probe and acquire position information of the end portion of the probe; and a controller configured to register the spectral image, the ultrasound image, and the color image on the basis of position information of the end portion of the probe and generate a three-dimensional image for three-dimensional diagnostic information for the object.

According to one aspect, the ultrasound image acquisition unit may include at least one or multiple ultrasound transducers disposed in an end portion of the probe, and the ultrasound transducer may perform scanning with respect to one area of the object.

According to one aspect, the ultrasound transducer may include a single or multiple ultrasound transducers and scanning of one area of the object may be performed by rotating the ultrasound transducer.

According to one aspect, a moving angle of the end portion of the probe may be determined in a direction perpendicular to a scanning direction of the ultrasound transducer on the basis of position information of the end portion of the probe, which is acquired through the inertia measurement unit.

According to one aspect, the three-dimensional diagnosis system may further includes a controller configured to control movement of the end portion of the probe and the ultrasound transducer, the controller may move the end portion of the probe in a direction perpendicular to the scanning direction of the ultrasound transducer, and the controller may control vertical movement of the end portion of the probe to be performed after the scanning of the ultrasound transducer with respect to the one area is completed, thereby automatically acquiring an ultrasound image.

According to one aspect, the controller may match the ultrasound image and the spectral image with respect to the object on the basis of position information of the end portion of the probe acquired through the inertia measurement unit.

According to one aspect, the controller may generate, on the basis of at least portion of a first ultrasound image and a second ultrasound image adjacent to each other and included in the multiple ultrasound images, a third ultrasound image between the first ultrasound image and the second ultrasound image by applying a generative adversarial network to the multiple ultrasound images acquired with respect to the one area.

According to one aspect, the controller may affine-transform a fourth ultrasound image on the basis of a transformation parameter acquired by applying a second learning model on the basis of a machine learning including a deep neural network to information of a position at which the multiple ultrasound images with respect to the one area are acquired, and the second learning model may be a learning model trained on the basis of training data in which position information of the end portion of the probe from which the ultrasound image was acquired is labeled as a transformation parameter for affine transformation.

According to one aspect, the ultrasound image acquisition unit further may further include: a pulser configured to control a pulse of an ultrasound signal measured from the ultrasound transducer; a receiver configured to receive the ultrasound signal; and a switch configured to open and close signal transfer between the ultrasound transducer and the receiver.

According to one aspect, the spectral image acquisition unit may include: a lens configured to collect light signal of the object; at least one optical fiber configured to transfer a light signal by the lens; and a bandpass filter array and a lens array configured to separate a light signal transferred by the optical fiber into light signals in multiple different wavelengths and receive the light signal.

According to one aspect, the spectral image acquisition unit may include: a lens configured to collect light signal of the object; at least one optical fiber configured to transfer a light signal by the lens; and a filter wheel including multiple filters configured to select a wavelength of a light signal transferred by the optical fiber and receive the light signal.

According to one aspect, the spectral image acquisition unit may include a light source member including white light and ultraviolet light, and the light source member may selectively emit the white light or the ultraviolet light toward the object.

According to one aspect, the three-dimensional diagnosis system may include a controller configured to apply a first learning model on the basis of machine learning including a convolution neural network to the multispectral image so as to output the multispectral image in which at least two different areas are distinguished in each wavelength-specific image of the multispectral image, and the learning model may be configured to generate an attention map by applying an attention mechanism of the neural network to each wavelength-specific image of the multispectral image and distinguish at least two different areas in each wavelength-specific image of the multispectral image on the basis of at least a portion of the attention map.

In accordance with another aspect, a three-dimensional diagnosis probe according to an embodiment may include: at least one optical element configured to acquire a spectral image with respect to surface information of an object; and at least one ultrasound transducer configured to be spaced apart from the optical element and acquire an ultrasound image with respect to depth information of the object, and the ultrasound transducer may rotate to perform scanning with respect to one area of the object.

According to one aspect, the three-dimensional diagnosis probe may further include at least one inertia measurement element configured to measure position information of an end portion of the probe.

According to one aspect, the end portion of the probe may move in a direction perpendicular to a scanning direction of the ultrasound transducer, and the ultrasound image and the spectral image with respect to the object may be matched on the basis of position information of the end portion of the probe acquired through the inertia measurement element.

### Advantageous Effects

A three-dimensional diagnosis system according to an example is advantageous in that not only surface information of a lesion, but also depth direction information thereof can be provided.

A three-dimensional diagnosis system according to an example is advantageous in that the distribution and invasive depth of a lesion can be inspected.

A three-dimensional diagnosis system according to an example is advantageous in that endoscopic optical imaging and ultrasound imaging are possible with the same wide field of view.

A three-dimensional diagnosis system according to an example is advantageous in that the lesion distribution of a tissue surface can be diagnosed and imaged through real-time spectral imaging and analysis.

A three-dimensional diagnosis system according to an example is advantageous in that high-frequency imaging is possible with the same field of view as optical imaging.

A three-dimensional diagnosis system according to an example is advantageous in that movements of an endoscope probe can be sensed.

A three-dimensional diagnosis system according to an example is advantageous in that ultrasound images can be stabilized in real time.

A three-dimensional diagnosis system according to an example is advantageous in that resolution can be improved by generating similar ultrasound images filling empty spaces between ultrasound images acquired based on acquired ultrasound images.

Advantageous effects of a three-dimensional diagnosis system according to an example are not limited to the above-mentioned advantageous effects, and other advantageous effects not mentioned herein will be clearly understood from the following description by those skilled in the art.

### Brief Description of Drawings

FIG. 1 illustrates a three-dimensional diagnosis system according to an example.
FIG. 2 is a schematic view of a three-dimensional diagnosis system configured in a snapshot mode according to an example.
FIG. 3 illustrates a probe of a three-dimensional diagnosis system according to an example.
FIG. 4 is a schematic view of a three-dimensional diagnosis system configured in a filter wheel mode according to another example.
FIG. 5 illustrates tissue to be scanned by using a three-dimensional diagnosis system according to an example.
FIG. 6 illustrates a spectral image of tissue acquired from a three-dimensional diagnosis system according to an example.
FIG. 7 illustrates an ultrasound image of tissue acquired from a three-dimensional diagnosis system according to an example.
FIG. 8 illustrates an image matching process of a three-dimensional diagnosis system according to an example.
FIG. 9 illustrates a learning model training method of a three-dimensional diagnosis system according to an example for outputting a multispectral image in which a lesion area is distinguished.
FIG. 10 illustrates a method of a learning model for generating an ultrasound image corresponding to an object between ultrasound images on the basis of multiple ultrasound images of a three-dimensional diagnosis system according to an example.
FIG. 11 illustrates a method of a learning model for affine transformation of an ultrasound image on the basis of multiple ultrasound images of a three-dimensional diagnosis system according to an example.
FIG. 12 illustrates a method for displaying by matching a three-dimensional ultrasound image and a stereoscopic image generated on the basis of a color image in a three-dimensional diagnosis system according to an example.

The following drawings attached to the disclosure illustrate a preferred example of the disclosure, and serve to further understand the technical idea of the disclosure together with a detailed description of the disclosure, so it should not be construed that the disclosure is limited to matters described in the drawings.

### Best Mode for Carrying out the Invention

Hereinafter, examples will be described in detail with reference to exemplary drawings. In allocating reference numerals to components of each drawing respectively, the same component may have the same reference numeral even though the same component is displayed on different drawings. Further, in the following description of examples, a detailed description of known functions and configurations incorporated herein will be omitted when it may make the subject matter of the present disclosure rather unclear.

Also, in describing the components of an example, there may be terms used like first, second, A, B, (a), and (b). These are solely for the purpose of differentiating one component from the other but not to imply or suggest the substances, order or sequence of the components. In the case that it is described that a certain structural element "is connected to", "is coupled to", or "is in contact with" another structural element, it should be interpreted that still another structural element may "be connected to", "be coupled to", or "be in contact with" the structural elements as well as that the certain structural element is directly connected to or is in direct contact with the another structural element.

The same name may be used to describe an element, which has the same function to an element included in one embodiment, in another embodiment. Unless described otherwise, a description which is given in one embodiment may be applicable to another embodiment, and a detailed description in an overlapping range may be omitted.

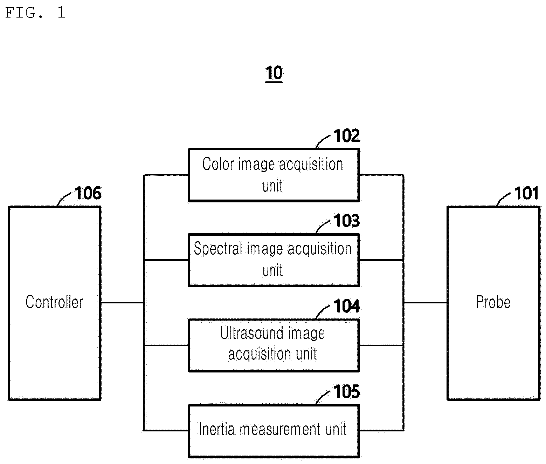

FIG. 1 illustrates a three-dimensional diagnosis system 10 according to an example.

Referring to FIG. 1, the three-dimensional diagnosis system 10 according to an example includes a probe 101, a color image acquisition unit 102, a spectral image acquisition unit 103, an ultrasound image acquisition unit 104, an inertia measurement unit 105, and a controller 106.

The probe 101 is configured to be positioned at a diagnosis object.

The color image acquisition unit 102 is configured to acquire a color image of an object through the probe 101.

The spectral image acquisition unit 103 is configured to acquire a spectral image with respect to surface information of an object through the probe 101.

The ultrasound image acquisition unit 104 is configured to acquire an ultrasound image with respect to depth information of an object through the probe 101.

The inertia measurement unit 105 detects movement of the probe 101.

The controller 106 may control driving of the probe 101, the color image acquisition unit 102, the spectral image acquisition unit 103, the ultrasound image acquisition unit 104, and the inertia measurement unit 105 described above. In addition, the controller 106 may process to receive and match an image or information acquired from each of the color image acquisition unit 102, the spectral image acquisition unit 103, the ultrasound image acquisition unit 104, and the inertia measurement unit 105, and control a final image according thereto to be displayed on a monitor or the like.

By using the three-dimensional diagnosis system 10 configured as described above according to an example, three-dimensional diagnosis for an object may be possible through a three-dimensional image generated by matching surface information and depth information of the object.

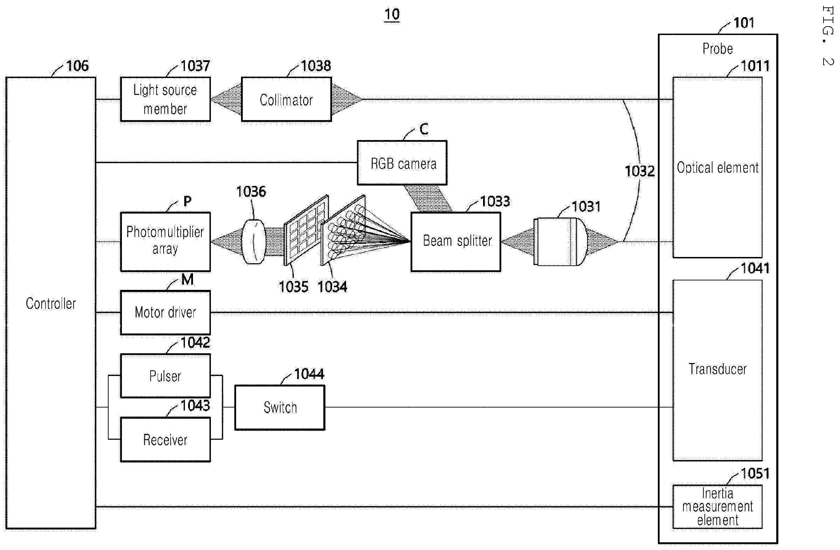

FIG. 2 is a schematic view of a three-dimensional diagnosis system 10 configured in a snapshot mode according to an example. In addition, FIG. 2 more specifically illustrates components of the probe 101, the color image acquisition unit 102, the spectral image acquisition unit 103, the ultrasound image acquisition unit 104, and the inertia measurement unit 105 of FIG. 1.

Referring to FIG. 2, the probe 101 may include an optical element 1011 and an ultrasound transducer 1041 and an inertia measurement element 1051 may be disposed at an end portion of the probe 101.

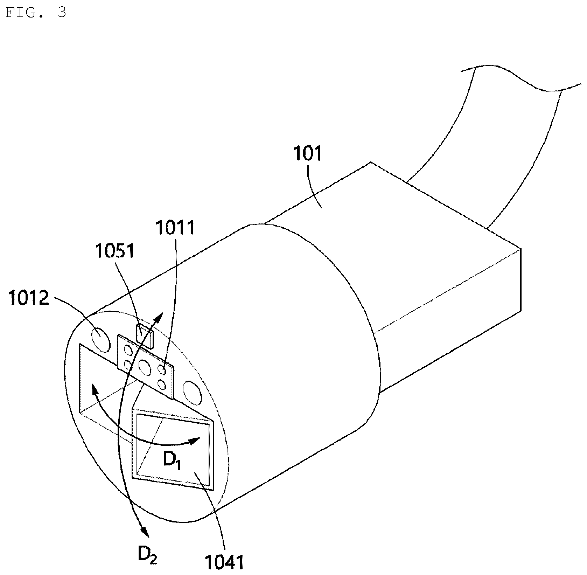

The probe 101 is more specifically illustrated in FIG. 3.

FIG. 3 illustrates a probe 101 of a three-dimensional diagnosis system according to an example.

Referring to FIG. 3, an optical element 1011, an ultrasound transducer 1041, and an inertia measurement element 1051 may be disposed at an end portion of the probe 101.

The optical element 1011 may be configured to acquire a spectral image with regard to surface information of an object. For this end, the optical element 1011 may include an imaging channel and a lighting channel. The imaging channel is a channel through which a light signal of an object is introduced and may be connected to an object lens 1031 through an optical fiber 1032. The lighting channel is a channel through which white light or ultraviolet light is emitted toward an object and may be connected to a light source member 1037 through the optical fiber 1032.

The ultrasound transducer 1041 may be disposed apart from the optical element 1011 and may be rotatably disposed in an end portion of the probe 101. The ultrasound transducer 1041 may perform scanning with respect to one area of an object while rotating in a first direction D1 so as to acquire an ultrasound image with respect to depth information of the object.

The inertia measurement element 1051 may be provided at one side of an end portion of the probe 101. The inertia measurement element 1051 may measure position information of the end portion of the probe 101.

In addition, a working channel 1012 may be provided at an end portion of the probe 101, and tools for performing an operation such as gripping and cutting an object may penetrate the working channel 1012.

The end portion of the probe 101 including components described above may be moved in a second direction D2.

Accordingly, the end portion of the probe 101 of the three-dimensional diagnosis system 10 according to an example may be moved in a direction perpendicular to the rotation direction of the ultrasound transducer 1041 while the ultrasound transducer 1041 rotates, so as to provide depth direction information as well as surface information of a lesion of an object.

Referring to FIG. 2 again, the color image acquisition unit 102 may include a camera for acquiring a color image of an object. For example, the camera may include an RGB camera C, a photomultiplier array P, and the like.

The color image acquisition unit 102 may acquire a color image of an object on the basis of a light signal received by the probe 101. For example, a spectral image, an ultrasound image, and angle information of the ultrasound image may be matched to the color image so as to finally acquire a three-dimensional image of the object.

The spectral image acquisition unit 103 may include the object lens 1031, the optical fiber 1032, a beam splitter 1033, a lens array 1034, a bandpass filter array 1035, a tube lens 1036, the RGB camera C, and the photomultiplier array P.

The object lens 1031 may be configured to collect a light signal of an object. For example, a fish-eye lens may be provided as the object lens 1031.

The optical fiber 1032 may transfer a light signal to the optical element 1011 or may transfer a light signal received from the optical element 1011 to the spectral image acquisition unit 103. The optical fiber 1032 may be connected to the optical element 1011 and connected to the object lens 1031 of the spectral image acquisition unit 103 or the collimator 1038. Here, the optical fiber 1032 connected to the object lens 1031 may be provided as multiple optical fiber bundles. In addition, the optical fiber 1032 connected to the collimator 1038 may be provided as a single optical fiber.

A light signal having penetrated the object lens 1031 may be transferred to each of the RGB camera C and the lens array 1034 by the beam splitter 1033. Furthermore, the beam splitter 1033 may be manually or automatically disposed at or removed from a light path depending on a purpose or purpose of use.

The lens array 1034 and the bandpass filter array 1035 may separate a light signal transferred by the optical fiber 1032 into light signals in multiple different wavelengths and receive the light signal. When a spectral image acquired from a light signal having penetrated through the lens array 1034 and the bandpass filter array 1035 to be separated may have a disadvantage of lowering resolution but may have an advantage of enabling spectral imaging in real time. In addition, the lens array 1034 and the bandpass filter array 1035 may be configured to be detachably attached.

A light signal having penetrated through the lens array 1034 and the bandpass filter array 1035 may penetrate through the tube lens 1036 to be transferred to the photomultiplier P.

The spectral image acquisition unit 103 may acquire a spectral image from a light signal of a surface of an object through the processes described above.

In addition, the spectral image acquisition unit 103 may further include the light source member 1037 and the collimator 1038.

The light source member 1037 may selectively output white light or ultraviolet light depending on a purpose. For example, the light source member 1037 may include a white LED and an ultraviolet (UV) LED.

The white light or ultraviolet light output from the light source member 1037 may penetrate at least one collimator 1038 to be emitted to an object through a single optical fiber 1032.

The spectral image acquisition unit 103, which is configured as described above, in a snapshot mode may acquire a spectral image with respect to surface information of an object through the probe 101 and selectively emit white light or ultraviolet light toward the object. In addition, it is possible to diagnose and image distribution of lesions on a tissue surface of an object through real-time spectral image and analysis.

The ultrasound image acquisition unit 104 may include the ultrasound transducer 1041, a pulser 1042, a receiver 1043, a switch 1044, and a motor driver **M.**

The ultrasound transducer 1041 may be rotatably disposed in an end portion of the probe 101. For example, the ultrasound transducer 1041 may be configured to be on transducer and transmit or receive an ultrasound signal to or from an object. The ultrasound transducer 1041 may perform scanning with respect to one area of an object while rotating in a first direction D1 inside the probe 101. Here, the first direction D1 may be a scanning direction of the ultrasound transducer 1041. In addition, one ultrasound transducer 1041 may be replaced with multiple ultrasound transducers.

The pulser 1042 may adjust a pulse of an ultrasound signal measured by the ultrasound transducer 1041.

The receiver 1043 may be configured to receive an ultrasound signal.

The switch 1044 may open or close signal transfer between the ultrasound transducer 1041 and the receiver 1043. For example, a T/R switch may be provided as the switch 1044. In addition, when multiple ultrasound transducers are used, multiple pulsers, receivers, and switches may be configured.

The motor driver M may transfer power to drive the ultrasound transducer 1041.

The ultrasound image acquisition unit 104 configured as described above may acquire an ultrasound image with respect to depth information of an object through the probe 101.

The inertia measurement unit 105 may include at least one inertia measurement element 1051.

The inertia measurement element 1051 may measure position information of the end portion of the probe 101. For example, the inertia measurement element 1051 may be provided as at least one IMU sensor.

A next movement angle of an end portion of the probe 101 may be determined by the controller 106 on the basis of position information of the end portion of the probe 101 acquired through the inertia measurement unit 1051. Here, the end portion of the probe 101 may be moved in a second direction D2 perpendicular to a first direction D1. That is, the end portion of the probe 101 may be moved in a direction perpendicular to a scanning direction of the ultrasound transducer 1041.

Movement of an endoscope probe 101 may be detected by the inertia measurement unit 105.

In addition, movement distance of the probe 101 may be calculated through a multiview stereoscopic distance measurement-based method using a real-time RGB image acquired when the end portion of the probe 101 moves while position information of the end portion of the probe 101 is simultaneously measured by using the inertia measurement unit 105.

The controller 106 may control movement of the probe 101 and driving of the color image acquisition unit 102, the spectral image acquisition unit 103, the ultrasound image acquisition unit 104, and the inertia measurement unit 105.

Particularly, in order to control movement of the end portion of the probe 101 and the ultrasound transducer 1041, the controller 106 may be electrically connected to the probe 101 as shown in FIG. 2, and may be electrically connected to the ultrasound transducer 1041 through the motor driver M.

The controller 106 may control the ultrasound transducer 1041 to rotate and move the end port of the probe 101 in a direction D2 perpendicular to the scanning direction D1 of the ultrasound transducer 1041.

Specifically, the controller 106 may rotate the ultrasound transducer 1041 in the first direction D1 to allow the ultrasound transducer 1041 to perform ultrasound scanning with regard to one area of an object. After the scanning with regard to one area is completed, the controller 106 may move the end portion of the probe 101 in the second direction D2 and then rotate the ultrasound transducer 1041 in the first direction D1 so as to perform ultrasound scanning with regard to next area. Therefore, an ultrasound image with respect to the object may be automatically acquired. In addition, an RGB multiview image may be continuously acquired during the scanning of the end portion of the probe 101 for acquiring a three-dimensional image. Accordingly, position information of the end portion of the probe 101 may be acquired through a stereoscopic-based depth measurement method by using the acquired RGB multiview image.

Simultaneously, since the controller 106 is electrically connected to the RGB camera C, a color image of an object may be received from a light signal of the object seen through the imaging channel of the probe 101 via the RGB camera C.

In addition, since the controller 106 is electrically connected to the light source member 1037, it may be controlled that at least one of the white LED or the ultraviolet LED is communicated with the collimator 1038 depending on user's purpose. That is, the controller 106 may selectively emit a light source through the lighting channel of the probe 101.

In addition, since the controller 106 is electrically connected to the photomultiplier array P, a reflection spectral image or fluorescence spectral image of an object acquired from a light signal of the object seen through the imaging channel of the probe 101 may be received.

In addition, since the controller 106 is electrically connected to the inertia measurement element 1051, position information of the end portion of the probe 101 acquired through the inertia measurement unit 105 may be received according to repetition of movement of the probe 101 and scanning of the ultrasound transducer 1041.

As a result, the controller 106 may process to match the above-described ultrasound image and the spectral image on the basis of the position information of the end portion of the probe 101.

Furthermore, the controller 106 may output a final matched three-dimensional image of the object or separately output, on display device such as a monitor, the color image, the spectral image, and the ultrasound image acquired from the color image acquisition unit 102, the spectral image acquisition unit 103, and the ultrasound image acquisition unit 104, respectively.

Therefore, by using the three-dimensional diagnosis system 10 configured in a snapshot mode according to an example, the spectral image having surface information of the object, multiple ultrasound images having depth information of the object, and angle information of each ultrasound image may be processed to be comprehensively matched in the color image such that a three-dimensional image with regard to the object may be finally acquired.

As a result, the distribution and invasion depth of lesions existing on the surface of the object and inside the object may be diagnosed in three dimensions.

Furthermore, a high-frequency image at the same viewing angle as an optical image is possible.

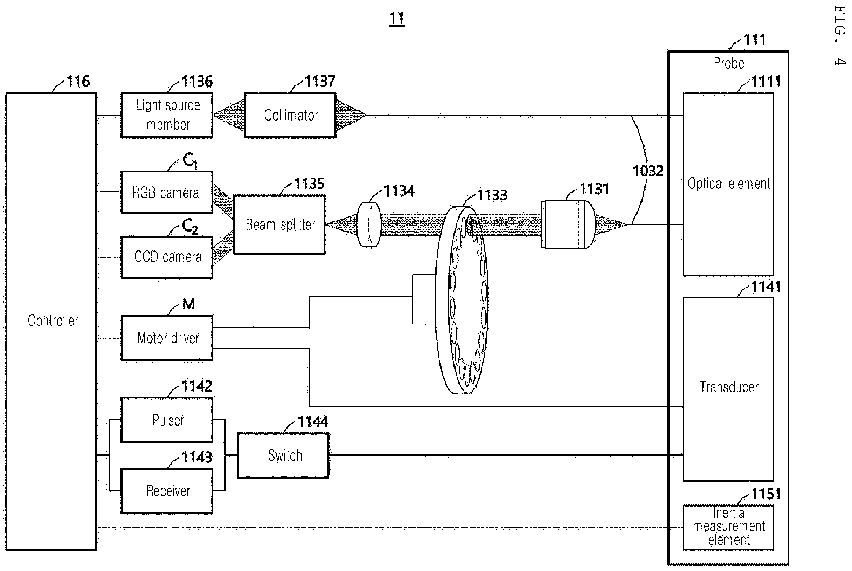

FIG. 4 is a schematic view of a three-dimensional diagnosis system 11 configured in a filter wheel mode according to an example. A spectral image acquisition unit 113 of the three-dimensional diagnosis system 11 in FIG. 4 may include different components from the spectral image acquisition unit 103 of the three-dimensional diagnosis system 10 in FIG. 2.

Referring to FIG. 4, the probe 111 may include an optical element 1111 and an ultrasound transducer 1141 and an inertia measurement element 1151 may be disposed at an end portion of the probe 111.

The probe 111 includes the same components as the above-described probe 101 of the three-dimensional diagnosis system 10 according to an example, thus the optical element 1111 of the three-dimensional diagnosis system 11 shown in FIG. 4 is the same as the optical element 1011 shown in FIG. 2, and a detailed description thereof will be omitted.

The color image acquisition unit 112 may include an RGB camera C1 and a CCD camera C2.

The color image acquisition unit 102 may acquire a color image of an object through the probe 101. In addition, for example, a spectral image, an ultrasound image, and angle information of the ultrasound image may be matched to the color image so as to finally acquire a three-dimensional image of the object.

The spectral image acquisition unit 113 may include an object lens 1131, an optical fiber 1132, a filter wheel 1133, a tube lens 1134, a beam splitter 1135, the RGB camera C1, and the CCD camera C2.

The object lens 1131 may be configured to collect a light signal of an object. For example, a fish-eye lens may be provided as the object lens 1131.

The optical fiber 1132 may transfer a light signal to the optical element 1111 or may transfer a light signal received from the optical element 1111 to the spectral image acquisition unit 113. The optical fiber 1132 may be connected to the optical element 1111 and connected to the object lens 1131 of the spectral image acquisition unit 113 or the collimator 10137. Here, the optical fiber 1132 connected to the object lens 1131 may be provided as multiple optical fiber bundles. In addition, the optical fiber 1132 connected to the collimator 1137 may be provided as a single optical fiber.

A light signal having penetrated the object lens 1131 may be transferred to the filter wheel 1133.

The filter wheel 1133 may include multiple filters for selecting a wavelength of a light signal transferred by the optical fiber 1132 to receive the light signal. In addition, the filter wheel 1133 may be connected to the motor driver M to be rotationally driven. The inclusion of the filter wheel 1133 may have a disadvantage that it may take longer than spectral imaging of the snapshot mode but have an advantage of acquiring a spectral image with higher resolution than that of the snapshot mode.

A light signal having penetrated through the bandpass filter wheel 1133 may penetrate through the tube lens 1134 to be transferred to the beam splitter 1135. Furthermore, a light signal may be transfer to each of the RGB camera C1 and the CCD camera C2 by the beam splitter 1135.

The spectral image acquisition unit 113 may acquire a spectral image from a light signal of a surface of an object through the processes described above.

In addition, the spectral image acquisition unit 113 may further include a light source member 1136 and a collimator 1137.

The light source member 1136 is the same as the light source member 1037 of FIG. 2, the collimator 1137 is the same as the collimator 1038 of FIG. **2****,** and thus a detailed description thereof will be omitted.

The spectral image acquisition unit 113 configured as described above may acquire a spectral image with respect to surface information of an object through the probe 111 and selectively emit white light or ultraviolet light toward the object. In addition, a spectral image having a higher resolution than the spectral image acquisition unit 103 in FIG. 2 may be acquired.

The ultrasound image acquisition unit 114 may include an ultrasound transducer 1141, a pulser 1142, a receiver 1143, a switch 1144, and a motor driver M.

The ultrasound image acquisition unit 114 includes the same components as the above-described ultrasound image acquisition unit 104 of the three-dimensional diagnosis system 10 according to an embodiment, thus the ultrasound transducer 1141, the pulser 1142, the receiver 1143, the switch 1144, and the motor driver M of the three-dimensional diagnosis system 11 in FIG. 4 are the same as the ultrasound transducer 1041, the pulser 1042, the receiver 1043, the switch 1044, and the motor driver M, respectively, and a detailed description thereof will be omitted.

The ultrasound image acquisition unit 114 configured as described above may acquire an ultrasound image with respect to depth information of an object through the probe 111.

The inertia measurement unit 115 may include at least one inertia measurement element 1151.

The inertia measurement unit 115 includes the same components as the above-described inertia measurement unit 115 of the three-dimensional diagnosis system 10 according to an embodiment, thus the inertia measurement element 1151 of the three-dimensional diagnosis system 11 in FIG. 4 is the same as the inertia measurement element 1051, and a detailed description thereof will be omitted.

A next movement angle of an end portion of the probe 111 may be determined by the controller 106 on the basis of position information of the end portion of the probe 111 acquired through the inertia measurement unit 1151.

The controller 116 may be electrically connected to the probe 111, the color image acquisition unit 112, the spectral image acquisition unit 113, the ultrasound image acquisition unit 114, and the inertia measurement unit 115, and may control movement of the probe 111 and driving of the color image acquisition unit 112, the spectral image acquisition unit 113, the ultrasound image acquisition unit 114, and the inertia measurement unit 115.

Similarly, the controller 116 performs the same functions through the same processes as that of the above-described controller 116 of the three-dimensional diagnosis system 10 according to an embodiment, and a detailed description thereof will be omitted.

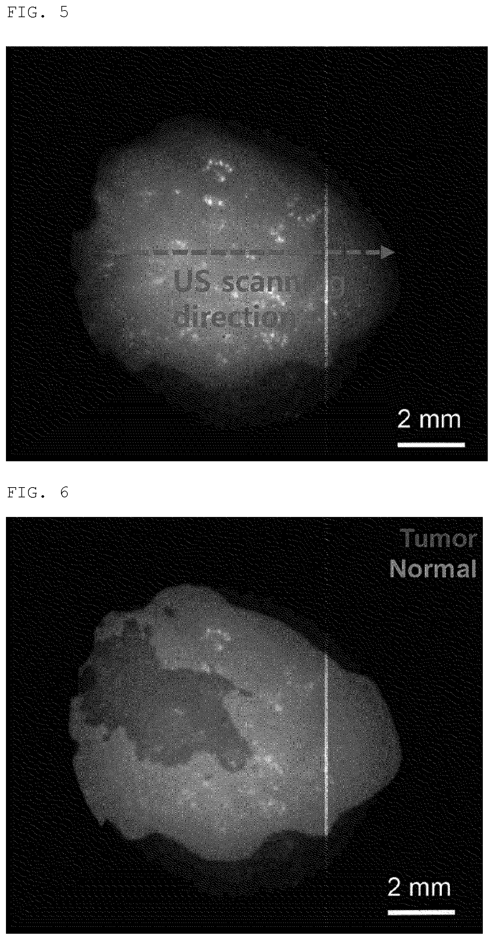

Hereinafter, referring to FIG. 5 to FIG. 8, a process of acquiring a three-dimensional image of an object by the three-dimensional diagnosis system 10, 11 will be described.

In addition, the object will be described using tissue in which cancer is distributed as an example.

FIG. 5 shows tissue scanned by using the three-dimensional diagnosis system 10, 11.

Referring to FIG. 5, a color image of the tissue may be acquired by using the three-dimensional diagnosis system 10, 11.

Simultaneously, the spectral image acquisition unit 103, 113 may irradiate the tissue with white light or ultraviolet light by using the light source member 1037, 1136 and transfer a light signal transferred from the tissue to the controller 106, 116.

Simultaneously, the ultrasound transducer 1041, 1141 may transmit an ultrasound signal into the tissue and transfer an ultrasound signal received from the tissue to the controller 106, 116 while rotating along a scanning direction shown in FIG. 5.

FIG. 6 illustrates a spectral image of tissue acquired from the three-dimensional diagnosis system 10, 11.

Referring to FIG. 6, a reflection spectral image or fluorescence spectral image with respect to surface information of the tissue may be acquired by using the three-dimensional diagnosis system 10, 11.

Here, as shown in FIG. 6, a cancer distribution area of the tissue surface may be determined through the spectral image.

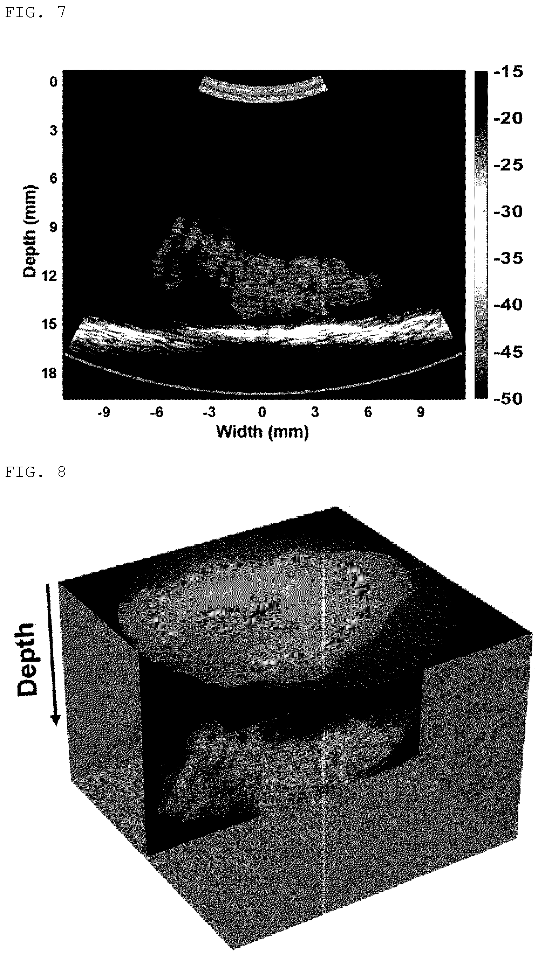

FIG. 7 illustrates an ultrasound image of tissue acquired from the three-dimensional diagnosis system 10, 11.

Referring to FIG. 7, an ultrasound image with respect to depth information of the tissue may be acquired by using the three-dimensional diagnosis system 10, 11.

Here, as shown in FIG. 7, a cancer distribution inside the tissue may be determined through the ultrasound image.

FIG. 8 illustrates a visual matching process of the three-dimensional diagnosis system 10, 11.

Movement of the ultrasound transducer 1041, 1141 and the probe 101, 111 is repeated by the controller 106, 116, and the inertia measurement element 1051, 1151 may simultaneously measure position information of the end portion of the probe 101 during each scan process.

Referring to FIG. 8, the controller 106, 116 may process to match the ultrasound image and the spectral image on the basis of the position information of the end portion of the probe 101, 111.

Therefore, by using the three-dimensional diagnosis system 10, 11, the spectral image having surface information of the object, the ultrasound image having depth information of the object, and angle information of the ultrasound image may be processed to be comprehensively matched in the color image such that a three-dimensional image with regard to the object may be finally acquired.

Accordingly, a target tissue surface and cancer distribution inside the tissue may be simultaneously diagnosed in three-dimensions.

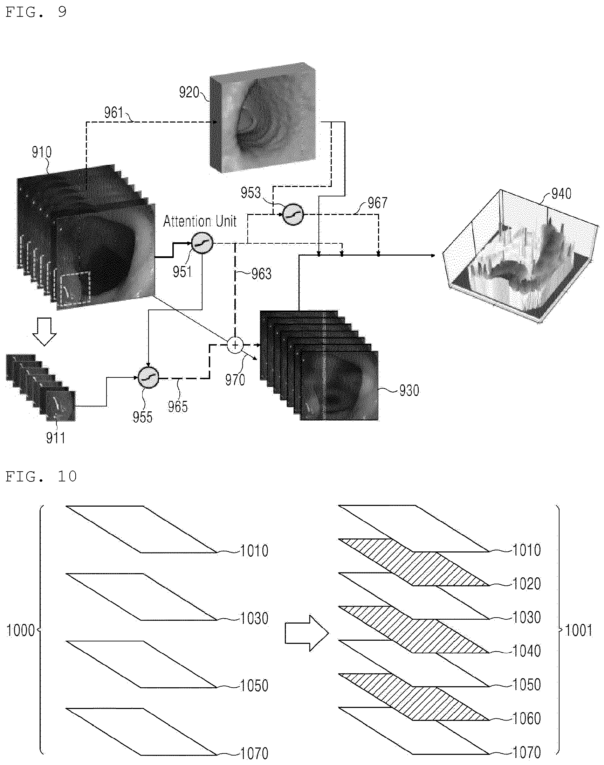

FIG. 9 illustrates a learning model training method of the three-dimensional diagnosis system 10, 11 for outputting a multispectral image 910 in which a lesion area is distinguished or a stereoscopic image 940 in which a lesion area is distinguished by the probability of distribution of malignant lesions.

A sensor unit of the three-dimensional diagnosis system 10, 11 may be the photomultiplier array P or the CCD camera C2 and may generate a multispectral image obtained by imaging optical signals of multiple different wavelengths for each wavelength, which are output from a spectral filtering component including the bandpass filter array 1035 or the beam splitter 1135.

A memory (not shown) may store a first learning model trained to output a multispectral image with at least two distinct regions which are different from each other in each wavelength-specific image of the multispectral image when the multispectral image is input.

The controller 106, 116 may apply the first learning model to the multispectral image 910 generated by the sensor unit 101, 111 to acquire the multispectral image 930 in which the lesion is marked differently from other areas.

The first learning model may include neural networks including three-dimensional convolution and may be a learning model to which an attention mechanism is applied. The attention may mean an operation performed to highlight main information by emphasizing some of input information. The first learning model may be trained to add attention to a lesion area in the multispectral image. The first learning model may include an attention gate 951, 953, 955 for providing the attention mechanism (for example, a mechanism for applying attention to an area having characteristic points of a lesion area).

In an example, the first learning model may include at least one feature map generated on the basis of the lesion image 911. The first learning model may apply attention with respect to the multispectral image on the basis of multiplying operation for each channel between the feature map and the multispectral image.

In an example, the first learning model may generate a gating signal 965, 963 on the basis of values inside the learning model, and the gating signal may be a weight for correcting a feature value.

In an example, the first learning model may signal such that feature points obtained from the multispectral image 910 and an image of a region of interest (ROI) such as a lesion area are input to at least one attention gate 951, 953, 955 and the attention gate 951, 953, 955 aggregates multiple feature points to output an optimum feature.

In an example, the attention gate 951, 953, 955 may generate scores corresponding to each feature and output an optimal feature after applying and summing a weighting parameter to multiple feature points on the basis of the generated score.

In an example, a second learning model may generate a malignant lesion distribution weight map 940 in which malignant lesions are distributed and weights are distinguished and displayed on a surface solid in the stereoscopic image 920 when the multispectral image 930 in which a lesion area is distinguished and a stereoscopic image 920 generated by a photometry stereo method in which a three-dimensional image is generated by calculating a surface normal and a reflection coefficient (albedo coefficient) in each pixel of multiple color images obtained by illuminating a region of interest of an object with white light from different directions are input.

FIG. 10 illustrates a method of a learning model for generating an ultrasound image corresponding to an object between ultrasound images on the basis of multiple ultrasound images of the three-dimensional diagnosis system 10, 11 according to an example.

A memory (not shown) may store the second learning model trained on the basis of a generative adversarial network for generating an ultrasound image between two different ultrasound images on the basis at least two different adjacent ultrasound images included in multiple ultrasound images when the multiple ultrasound images are input.

In an example, a third learning model may include multiple neural networks including three-dimensional convolution. The third learning model may include a generation model including a neural network trained to generate an ultrasound image 1020, 2040, 1060 between adjacent ultrasound images 1010, 1030, 1050, 1070 by receiving multiple ultrasound images 1000, and a discrimination model including a neural network trained to discriminate whether the generated ultrasound image 1020, 2040, 1060 is a real ultrasound image or generated ultrasound image. In an embodiment, the generation model may include down-sampling for extracting feature points of an input ultrasound image set for transformation and up-sampling for reconstructing an image on the basis of the extracted feature points, and the down-sampling and the up-sampling may include a convolution layer. Information of high-frequency components may be shared by using a skip connection in order to prevent loss of information in an original ultrasound image, and for example, information of a specific layer of a down-sampling process may be connected to information of a specific layer of an up-sampling process.

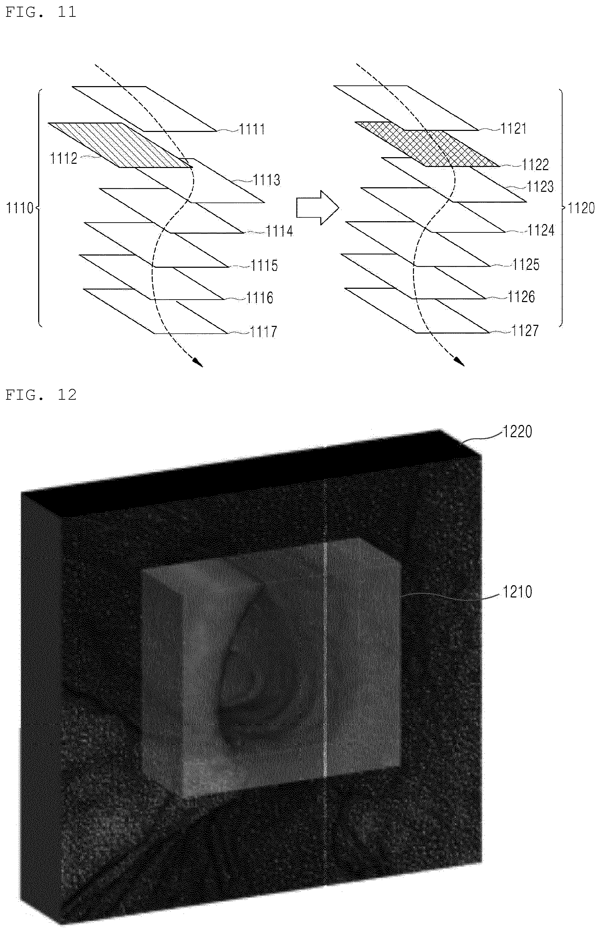

FIG. 11 illustrates a method of a learning model for affine transformation of an ultrasound image on the basis of multiple ultrasound images of the three-dimensional diagnosis system 10, 11 according to an example.

The three-dimensional diagnosis system 10, 11 may acquire multiple ultrasound images 1110 according to movement of the end portion of the probe 101, 111, a partial image 1112 thereof may be acquired at another area of the object compared with other ultrasound images 1111, 1113-1117 acquired at a stable moving position of the end portion of the probe 101, 111 due to local rapid movement or vibration of the end portion of the probe 101, 111, and in this case, an image quality of the three-dimensional ultrasound image may be damaged.

In an example, a memory (not shown) may store a fourth learning model trained to output the multiple ultrasound images 1111-1117 as affine deformable parameters (pitch, yaw, roll) when information of a position at which multiple ultrasound images 1111-1117 are acquired, on the basis of the inertia measurement element with respect to one area of the object is input.

In an embodiment, a memory (not shown) may store a fourth learning model trained to output the multiple ultrasound images 1111-1117 as affine deformable parameters (pitch, yaw, roll) or multiple ultrasound images 1121-1127 obtained by affine deforming the multiple ultrasound images 1111-1117 when information of a position at which multiple ultrasound images 1111-1117 are acquired, on the basis of the inertia measurement element with respect to one area of the object and the multiple ultrasound images 1111-1117, are input.

In an example, a fourth learning model may include a neural network including three-dimensional convolution.

The conventional ultrasound image stabilization technique has been difficult to apply in real time due to the amount of computation on the basis of a method of finding a parameter that maximizes the value of a function in a function for examining the similarity between ultrasound images, but the method has an effect that stabilization of ultrasound images may be performed in real time.

FIG. 12 illustrates a method for displaying by matching a stereoscopic image generated on the basis of a color image acquired from the object to three-dimensional ultrasound image generated on the basis of multiple ultrasound images acquired from the object in the three-dimensional diagnosis system 10, 11.

The stereoscopic image may be a three-dimensional surface image generated for an area where a color image was acquired on the basis of the surface normal and reflection coefficient (albedo coefficient) calculated in each pixel of color images acquired by illuminating an object with white light from different directions on the basis of the photometric stereo imaging method.

In an example, the three-dimensional diagnosis system 10, 11 may calculate a transformation parameter for registration of a three-dimensional ultrasound image and a three-dimensional surface image on the basis of information of a position at which each ultrasound image of the three-dimensional ultrasound image is acquired and position information of a color image.

In an example, the three-dimensional diagnosis system 10, 11 may display overlays by matching and blending the three-dimensional ultrasound image and the three-dimensional surface image through application of a scale invariant feature transform algorithm on the basis of image similarity between the ultrasound images and the three-dimensional surface image.

### [Reference Numerals]

10: three-dimensional diagnosis system
101: probe
1011: optical element
1012: working channel
102: color image acquisition unit
103: spectral image acquisition unit
1031: objective lens
1032: optical fiber
1033: beam splitter
1034: lens array
1035: bandpass filter array
1036: tube lens
1037: light source member
1038: collimator
104: ultrasound image acquisition unit
1041: ultrasound transducer
1042: pulser
1043: receiver
1044: switch
105: inertia measurement unit
1051: inertia measurement element
106: controller
910: multispectral image
911: lesion image
920, 1210: surface stereoscopic image
951, 953, 955: attention gate
1220: three-dimensional ultrasound image

## Claims

1. A three-dimensional diagnosis system comprising:
a probe (101; 111) configured to be positioned at an object to be diagnosed;
a spectral image acquisition unit (103) configured to acquire a spectral image with respect to surface information of the object through the probe (101; 111);
an ultrasound image acquisition unit (104) configured to acquire an ultrasound image with respect to depth information of an object through the probe (101; 111);
a color image acquisition unit (102) configured to acquire a color image of the object through the probe (101; 111);
an inertia measurement unit (105) configured to detect movement of the probe (101, 111) and acquire position information of the end portion of the probe (101, 111); and
a controller (106) configured to register the spectral image, the ultrasound image, and the color image on the basis of position information of the end portion of the probe (101, 111), and generate a three-dimensional image for three-dimensional diagnostic information for the object.

2. The three-dimensional diagnosis system of claim 1,
wherein the ultrasound image acquisition unit (104) comprises at least single-element or multi-element ultrasound transducers (1041; 1141) disposed in an end portion of the probe (101; 111), and
wherein the ultrasound transducer (1041; 1141) performs scanning with respect to one area of the object.

3. The three-dimensional diagnosis system of claim 2,
wherein the ultrasound transducer (1041; 1141) comprises a single-element or multi-element ultrasound transducers (1041; 1141), and scanning for one area of the object is performed by rotating the ultrasound transducer.

4. The three-dimensional diagnosis system of claim 2 or claim 3,
wherein a moving angle of the end portion of the probe (101; 111) is determined in a direction perpendicular to a scanning direction of the ultrasound transducer (1041; 1141) on the basis of position information of the end portion of the probe (101; 111), which is acquired through the inertia measurement unit (105).

5. The three-dimensional diagnosis system of claim 4,
wherein the controller (106) moves the end portion of the probe (101; 111) in a direction perpendicular to the scanning direction of the ultrasound transducer (1041; 1141), and
wherein the controller (106) controls vertical movement of the end portion of the probe (101; 111) to be performed after the scanning of the ultrasound transducer (1041; 1141) with respect to the one area is completed, thereby automatically acquiring an ultrasound image.

6. The three-dimensional diagnosis system of claim 5,
wherein the controller (106) registers the ultrasound image and the spectral image with respect to the object on the basis of position information of the end portion of the probe (101; 111) acquired through the inertia measurement unit (105).

7. The three-dimensional diagnosis system of claim 2,
wherein the ultrasound image acquisition unit (104) further comprises:
a pulser (1042) configured to control a pulse of an ultrasound signal from the ultrasound transducer (1041; 1141);
a receiver (1043) configured to receive the ultrasound signal; and
a switch (1044) configured to open and close signal transfer between the ultrasound transducer (1041; 1141) and the receiver.

8. The three-dimensional diagnosis system of claim 1,
wherein the spectral image acquisition unit (103) comprises:
a lens (1031; 1131) configured to collect a light signal of the object;
at least one optical fiber (1032) configured to transfer a light signal by the lens (1031; 1131); and
a bandpass filter array (1035; 1135) and a lens array (1034; 1134) configured to separate a light signal transferred by the optical fiber (1032) into light signals in multiple different wavelengths and receive the light signal.

9. The three-dimensional diagnosis system of claim 1,
wherein the spectral image acquisition unit (103) comprises:
a lens (1031; 1131) configured to collect light signal of the object;
at least one optical fiber (1032) configured to transfer a light signal by the lens (1031; 1131); and
a filter wheel (1133) comprising multiple filters configured to select a wavelength of a light signal transferred by the optical fiber and receive the light signal.

10. The three-dimensional diagnosis system of claim 1,
wherein the spectral image acquisition unit (103) comprises:
a lens (1031; 1131) configured to collect light signal of the object;
at least one optical fiber (1032) configured to transfer a light signal by the lens (1031; 1131);
a spectral filtering component (1034; 1035; 1133) configured to separate a light signal transferred by the optical fiber (1032) into light signals in multiple different wavelengths; and
a sensor unit (P; C1; C2) configured to generate a multispectral image obtained by imaging light signals in multiple different wavelengths for each wavelength.

11. The three-dimensional diagnosis system of claim 10, wherein the controller (106) applies a first learning model on the basis of machine learning including a convolution neural network to the multispectral image so as to output the multispectral image in which at least two different areas are distinguished in each wavelength-specific image of the multispectral image, and
wherein the learning model is configured to generate an attention map by applying an attention mechanism of the neural network to each wavelength-specific image of the multispectral image and distinguish at least two different areas in each wavelength-specific image of the multispectral image on the basis of at least a portion of the attention map.

12. The three-dimensional diagnosis system of claim 5,
wherein the controller (106) generates, on the basis of at least portion of a first ultrasound image and a second ultrasound image adjacent to each other and included in the multiple ultrasound images, a third ultrasound image between the first ultrasound image and the second ultrasound image by applying a generative adversarial network to the multiple ultrasound images acquired with respect to the one area.

13. The three-dimensional diagnosis system of claim 5,
wherein the controller affine-transforms a fourth ultrasound image on the basis of a transformation parameter acquired by applying a second learning model on the basis of a machine learning including a deep neural network to information of a position at which the multiple ultrasound images with respect to the one area are acquired, and
wherein the second learning model is a learning model trained on the basis of training data in which position information of the end portion of the probe (101; 111) from which the ultrasound image was acquired is labeled as a transformation parameter for affine transformation.

14. The three-dimensional diagnosis system of claim 5,
wherein the controller (106) acquires multiple color images on the basis of a light source which illuminates the object from multiple directions, and matches a stereoscopic image generated on the basis of a surface normal and a reflection coefficient of the multiple color images to the ultrasound image with respect to the object on the basis of image similarity.

## Patentansprüche

1. Ein System für dreidimensionale Diagnose, das Folgendes beinhaltet:
eine Sonde (101; 111), die konfiguriert ist, um an einem zu diagnostizierenden Objekt positioniert zu werden;
eine Spektralbilderfassungseinheit (103), die konfiguriert ist, um ein Spektralbild in Bezug auf Oberflächeninformationen des Objekts durch die Sonde (101; 111) zu erfassen;
eine Ultraschallbilderfassungseinheit (104), die konfiguriert ist, um ein Ultraschallbild in Bezug auf Tiefeninformationen eines Objekts durch die Sonde (101; 111) zu erfassen;
eine Farbbilderfassungseinheit (102), die konfiguriert ist, um ein Farbbild des Objekts durch die Sonde (101; 111) zu erfassen;
eine Trägheitsmesseinheit (105), die konfiguriert ist, um eine Bewegung der Sonde (101, 111) zu detektieren und Positionsinformationen des Endabschnitts der Sonde (101, 111) zu erfassen; und
eine Steuervorrichtung (106), die konfiguriert ist, um das Spektralbild, das Ultraschallbild und das Farbbild auf der Basis von Positionsinformationen des Endabschnitts der Sonde (101, 111) in Register zu bringen und ein dreidimensionales Bild für dreidimensionale Diagnoseinformationen für das Objekt zu erzeugen.

2. System für dreidimensionale Diagnose gemäß Anspruch 1,
wobei die Ultraschallbilderfassungseinheit (104) mindestens Einzelelement- oder Mehrelement-Ultraschallwandler (1041; 1141) beinhaltet, die in einem Endabschnitt der Sonde (101; 111) angeordnet sind, und
wobei der Ultraschallwandler (1041; 1141) ein Scannen in Bezug auf einen Bereich des Objekts durchführt.

3. System für dreidimensionale Diagnose gemäß Anspruch 2,
wobei der Ultraschallwandler (1041; 1141) einen Einzelelement- oder Mehrelement-Ultraschallwandler (1041; 1141) beinhaltet und das Scannen für einen Bereich des Objekts durch Drehen des Ultraschallwandlers durchgeführt wird.

4. System für dreidimensionale Diagnose gemäß Anspruch 2 oder Anspruch 3, wobei ein Bewegungswinkel des Endabschnitts der Sonde (101; 111) auf der Basis von Positionsinformationen des Endabschnitts der Sonde (101; 111), die durch die Trägheitsmesseinheit (105) erfasst werden, in einer zu einer Scanrichtung des Ultraschallwandlers (1041; 1141) senkrechten Richtung bestimmt wird.

5. System für dreidimensionale Diagnose gemäß Anspruch 4,
wobei die Steuervorrichtung (106) den Endabschnitt der Sonde (101; 111) in einer zu der Scanrichtung des Ultraschallwandlers (1041; 1141) senkrechten Richtung bewegt, und
wobei die Steuervorrichtung (106) eine vertikale Bewegung des Endabschnitts der Sonde (101; 111) steuert, die durchzuführen ist, nachdem das Scannen des Ultraschallwandlers (1041; 1141) in Bezug auf den einen Bereich abgeschlossen ist, wodurch automatisch ein Ultraschallbild erfasst wird.

6. System für dreidimensionale Diagnose gemäß Anspruch 5,
wobei die Steuervorrichtung (106) das Ultraschallbild und das Spektralbild in Bezug auf das Objekt auf der Basis von Positionsinformationen des Endabschnitts der Sonde (101; 111), die durch die Trägheitsmesseinheit (105) erfasst werden, in Register bringt.

7. System für dreidimensionale Diagnose gemäß Anspruch 2,
wobei die Ultraschallbilderfassungseinheit (104) ferner Folgendes beinhaltet:
einen Impulsgeber (1042), der konfiguriert ist, um einen Impuls eines Ultraschallsignals von dem Ultraschallwandler (1041; 1141) zu steuern;
einen Empfänger (1043), der konfiguriert ist, um das Ultraschallsignal zu empfangen; und
einen Schalter (1044), der konfiguriert ist, um eine Signalübertragung zwischen dem Ultraschallwandler (1041; 1141) und dem Empfänger zu öffnen und zu schließen.

8. System für dreidimensionale Diagnose gemäß Anspruch 1,
wobei die Spektralbilderfassungseinheit (103) Folgendes beinhaltet:
eine Linse (1031; 1131), die konfiguriert ist, um ein Lichtsignal des Objekts zu sammeln;
mindestens eine optische Faser (1032), die konfiguriert ist, um ein Lichtsignal durch die Linse (1031; 1131) zu übertragen; und
eine Bandpassfilteranordnung (1035; 1135) und eine Linsenanordnung (1034; 1134),
die konfiguriert sind, um ein durch die optische Faser (1032) übertragenes Lichtsignal in Lichtsignale in mehreren unterschiedlichen Wellenlängen zu trennen und das Lichtsignal zu empfangen.

9. System für dreidimensionale Diagnose gemäß Anspruch 1,
wobei die Spektralbilderfassungseinheit (103) Folgendes beinhaltet:
eine Linse (1031; 1131), die konfiguriert ist, um ein Lichtsignal des Objekts zu sammeln;
mindestens eine optische Faser (1032), die konfiguriert ist, um ein Lichtsignal durch die Linse (1031; 1131) zu übertragen; und
ein Filterrad (1133), das mehrere Filter beinhaltet, die konfiguriert sind, um eine Wellenlänge eines durch die optische Faser übertragenen Lichtsignals auszuwählen und das Lichtsignal zu empfangen.

10. System für dreidimensionale Diagnose gemäß Anspruch 1,
wobei die Spektralbilderfassungseinheit (103) Folgendes beinhaltet:
eine Linse (1031; 1131), die konfiguriert ist, um ein Lichtsignal des Objekts zu sammeln;
mindestens eine optische Faser (1032), die konfiguriert ist, um ein Lichtsignal durch die Linse (1031; 1131) zu übertragen;
eine Spektralfilterkomponente (1034; 1035; 1133), die konfiguriert ist, um ein durch die optische Faser (1032) übertragenes Lichtsignal in Lichtsignale in mehreren unterschiedlichen Wellenlängen zu trennen; und
eine Sensoreinheit (P; C1; C2), die konfiguriert ist, um ein multispektrales Bild zu erzeugen, das durch Abbilden von Lichtsignalen in mehreren unterschiedlichen Wellenlängen für jede Wellenlänge erhalten wird.

11. System für dreidimensionale Diagnose gemäß Anspruch 10, wobei die Steuervorrichtung (106) ein erstes Lernmodell auf der Basis von maschinellem Lernen, das ein neuronales Faltungsnetzwerk umfasst, auf das multispektrale Bild anwendet, um das multispektrale Bild auszugeben, in dem mindestens zwei unterschiedliche Bereiche in jedem wellenlängenspezifischen Bild des multispektralen Bildes unterschieden werden, und
wobei das Lernmodell konfiguriert ist, um durch Anwenden eines Aufmerksamkeitsmechanismus des neuronalen Netzwerks auf jedes wellenlängenspezifische Bild des multispektralen Bildes eine Aufmerksamkeitskarte zu erzeugen und mindestens zwei unterschiedliche Bereiche in jedem wellenlängenspezifischen Bild des multispektralen Bildes auf der Basis von mindestens einem Abschnitt der Aufmerksamkeitskarte zu unterscheiden.

12. System für dreidimensionale Diagnose gemäß Anspruch 5,
wobei die Steuervorrichtung (106) auf der Basis von mindestens einem Abschnitt eines ersten Ultraschallbildes und eines zweiten Ultraschallbildes, die aneinander angrenzen und in den mehreren Ultraschallbildern enthalten sind, durch Anwenden eines generativen adversarialen Netzwerks auf die mehreren Ultraschallbilder, die in Bezug auf den einen Bereich erfasst werden, ein drittes Ultraschallbild zwischen dem ersten Ultraschallbild und dem zweiten Ultraschallbild erzeugt.

13. System für dreidimensionale Diagnose gemäß Anspruch 5,
wobei die Steuervorrichtung auf der Basis eines Transformationsparameters, der durch Anwenden eines zweiten Lernmodells auf der Basis von maschinellem Lernen, das ein tiefes neuronales Netzwerk umfasst, auf Informationen einer Position, an der die mehreren Ultraschallbilder in Bezug auf den einen Bereich erfasst werden, erfasst wird, ein viertes Ultraschallbild affin transformiert und
wobei das zweite Lernmodell ein Lernmodell ist, das auf der Basis von Trainingsdaten trainiert wird, in denen Positionsinformationen des Endabschnitts der Sonde (101; 111), von der das Ultraschallbild erfasst wurde, als ein Transformationsparameter für affine Transformation gekennzeichnet sind.

14. System für dreidimensionale Diagnose gemäß Anspruch 5,
wobei die Steuervorrichtung (106) mehrere Farbbilder auf der Basis einer Lichtquelle erfasst, die das Objekt aus mehreren Richtungen beleuchtet, und ein stereoskopisches Bild, das auf der Basis einer Oberflächennormalen und eines Reflexionskoeffizienten der mehreren Farbbilder erzeugt wird, mit dem Ultraschallbild in Bezug auf das Objekt auf der Basis von Bildähnlichkeit abgleicht.

## Revendications

1. Un système de diagnostic en trois dimensions comprenant :
une sonde (101 ; 111) configurée pour être positionnée au niveau d'un objet devant être diagnostiqué ;
une unité d'acquisition d'image spectrale (103) configurée pour acquérir une image spectrale par rapport à des informations de surface de l'objet par l'intermédiaire de la sonde (101 ; 111) ;
une unité d'acquisition d'image ultrasonore (104) configurée pour acquérir une image ultrasonore par rapport à des informations de profondeur d'un objet par l'intermédiaire de la sonde (101 ; 111) ;
une unité d'acquisition d'image en couleur (102) configurée pour acquérir une image en couleur de l'objet par l'intermédiaire de la sonde (101 ; 111) ;
une unité de mesure d'inertie (105) configurée pour détecter un mouvement de la sonde (101, 111) et acquérir des informations de position de la partie d'extrémité de la sonde (101, 111) ; et
un dispositif de commande (106) configuré pour enregistrer l'image spectrale, l'image ultrasonore, et l'image en couleur sur la base d'informations de position de la partie d'extrémité de la sonde (101, 111), et générer une image en trois dimensions pour des informations de diagnostic en trois dimensions pour l'objet.

2. Le système de diagnostic en trois dimensions de la revendication 1,
dans lequel l'unité d'acquisition d'image ultrasonore (104) comprend au moins des transducteurs ultrasonores à élément unique ou à éléments multiples (1041 ; 1141) disposés dans une partie d'extrémité de la sonde (101 ; 111), et
dans lequel le transducteur ultrasonore (1041 ; 1141) effectue un balayage par rapport à une zone de l'objet.

3. Le système de diagnostic en trois dimensions de la revendication 2,
dans lequel le transducteur ultrasonore (1041 ; 1141) comprend un transducteur ultrasonore à élément unique ou à éléments multiples (1041 ; 1141), et le balayage pour une zone de l'objet est effectué par rotation du transducteur ultrasonore.

4. Le système de diagnostic en trois dimensions de la revendication 2 ou de la revendication 3,
dans lequel un angle de mouvement de la partie d'extrémité de la sonde (101 ; 111) est déterminé dans une direction perpendiculaire à une direction de balayage du transducteur ultrasonore (1041 ; 1141) sur la base d'informations de position de la partie d'extrémité de la sonde (101 ; 111), qui sont acquises par l'intermédiaire de l'unité de mesure d'inertie (105).

5. Le système de diagnostic en trois dimensions de la revendication 4,
dans lequel le dispositif de commande (106) meut la partie d'extrémité de la sonde (101 ; 111) dans une direction perpendiculaire à la direction de balayage du transducteur ultrasonore (1041 ; 1141), et
dans lequel le dispositif de commande (106) commande un mouvement vertical de la partie d'extrémité de la sonde (101 ; 111) devant être effectué après que le balayage du transducteur ultrasonore (1041 ; 1141) par rapport à cette zone a été accompli, acquérant ainsi automatiquement une image ultrasonore.

6. Le système de diagnostic en trois dimensions de la revendication 5,
dans lequel le dispositif de commande (106) enregistre l'image ultrasonore et l'image spectrale par rapport à l'objet sur la base d'informations de position de la partie d'extrémité de la sonde (101 ; 111) acquises par l'intermédiaire de l'unité de mesure d'inertie (105).

7. Le système de diagnostic en trois dimensions de la revendication 2,
dans lequel l'unité d'acquisition d'image ultrasonore (104) comprend en outre :
un pulseur (1042) configuré pour commander une impulsion d'un signal ultrasonore provenant du transducteur ultrasonore (1041 ; 1141) ;
un récepteur (1043) configuré pour recevoir le signal ultrasonore ; et
un interrupteur (1044) configuré pour ouvrir et fermer un transfert de signal entre le transducteur ultrasonore (1041 ; 1141) et le récepteur.

8. Le système de diagnostic en trois dimensions de la revendication 1,
dans lequel l'unité d'acquisition d'image spectrale (103) comprend :
une lentille (1031 ; 1131) configurée pour recueillir un signal lumineux de l'objet ;
au moins une fibre optique (1032) configurée pour transférer un signal lumineux par la lentille (1031 ; 1131) ; et
un groupement de filtres passe-bande (1035 ; 1135) et un groupement de lentilles (1034 ; 1134) configurés pour séparer un signal lumineux transféré par la fibre optique (1032) en signaux lumineux dans de multiples longueurs d'onde différentes et recevoir le signal lumineux.

9. Le système de diagnostic en trois dimensions de la revendication 1,
dans lequel l'unité d'acquisition d'image spectrale (103) comprend :
une lentille (1031 ; 1131) configurée pour recueillir un signal lumineux de l'objet ;
au moins une fibre optique (1032) configurée pour transférer un signal lumineux par la lentille (1031 ; 1131) ; et
une roue à filtres (1133) comprenant de multiples filtres configurés pour sélectionner une longueur d'onde d'un signal lumineux transféré par la fibre optique et recevoir le signal lumineux.

10. Le système de diagnostic en trois dimensions de la revendication 1,
dans lequel l'unité d'acquisition d'image spectrale (103) comprend :
une lentille (1031 ; 1131) configurée pour recueillir un signal lumineux de l'objet ;
au moins une fibre optique (1032) configurée pour transférer un signal lumineux par la lentille (1031 ; 1131) ;
un composant de filtrage spectral (1034 ; 1035 ; 1133) configuré pour séparer un signal lumineux transféré par la fibre optique (1032) en signaux lumineux dans de multiples longueurs d'onde différentes ; et
une unité de capteur (P ; C1 ; C2) configurée pour générer une image multispectrale obtenue par imagerie de signaux lumineux dans de multiples longueurs d'onde différentes pour chaque longueur d'onde.

11. Le système de diagnostic en trois dimensions de la revendication 10, dans lequel le dispositif de commande (106) applique un premier modèle d'apprentissage sur la base d'un apprentissage machine incluant un réseau neuronal convolutif à l'image multispectrale de façon à délivrer en sortie l'image multispectrale dans laquelle au moins deux zones différentes sont distinguées dans chaque image spécifique à la longueur d'onde de l'image multispectrale, et
dans lequel le modèle d'apprentissage est configuré pour générer une carte d'attention par application d'un mécanisme d'attention du réseau neuronal à chaque image spécifique à la longueur d'onde de l'image multispectrale et distinguer au moins deux zones différentes dans chaque image spécifique à la longueur d'onde de l'image multispectrale sur la base d'au moins une partie de la carte d'attention.

12. Le système de diagnostic en trois dimensions de la revendication 5,
dans lequel le dispositif de commande (106) génère, sur la base d'au moins une partie d'une première image ultrasonore et d'une deuxième image ultrasonore adjacentes l'une à l'autre et incluses dans les multiples images ultrasonores, une troisième image ultrasonore entre la première image ultrasonore et la deuxième image ultrasonore par application d'un réseau antagoniste génératif aux multiples images ultrasonores acquises par rapport à cette zone.

13. Le système de diagnostic en trois dimensions de la revendication 5,
dans lequel le dispositif de commande effectue une transformation affine d'une quatrième image ultrasonore sur la base d'un paramètre de transformation acquis par application d'un deuxième modèle d'apprentissage sur la base d'un apprentissage machine incluant un réseau neuronal profond à des informations d'une position à laquelle les multiples images ultrasonores par rapport à cette zone sont acquises, et dans lequel le deuxième modèle d'apprentissage est un modèle d'apprentissage entraîné sur la base de données d'entraînement dans lesquelles des informations de position de la partie d'extrémité de la sonde (101 ; 111) à partir de laquelle l'image ultrasonore a été acquise sont marquées comme paramètre de transformation pour une transformation affine.

14. Le système de diagnostic en trois dimensions de la revendication 5,
dans lequel le dispositif de commande (106) acquiert de multiples images en couleur sur la base d'une source de lumière qui éclaire l'objet à partir de multiples directions, et fait correspondre une image stéréoscopique générée sur la base d'une normale de surface et d'un coefficient de réflexion des multiples images en couleur à l'image ultrasonore par rapport à l'objet sur la base d'une similarité d'image.
